Europäisches Patentamt

European Patent Office

Office européen des brevets

(19)

(11) Numéro de publication: **0 117 766**
**A1**

(12)

# DEMANDE DE BREVET EUROPEEN

(21) Numéro de dépôt: **84400022.4**

(22) Date de dépôt: **06.01.84**

(51) Int. Cl.³: **A 01 G 31/02**, A 01 G 9/10

(30) Priorité: **07.01.83 FR 8300180**

(43) Date de publication de la demande: **05.09.84**
**Bulletin 84/36**

(84) Etats contractants désignés: **AT BE CH DE FR GB IT LI LU NL SE**

(71) Demandeur: **CREATIONS CHALLET HERAUT S.a.r.l., Nuaillé, F-49340 Trementines (FR)**

(72) Inventeur: **Challet, Paul, "Le Courtil" Nuaillé, F-49340 Trementines (FR)**
Inventeur: **Brochard, Patrice, 24, Boulevard Victor-Hugo, F-49300 Cholet (FR)**
Inventeur: **Cailleton, Jacques, Rue Joseph Foyer, F-49360 Maulevrier (FR)**

(74) Mandataire: **Bruder, Michel, 10 rue de la Pépinière, F-75008 Paris (FR)**

(54) **Substrat de culture pour les cellules, tissus, organes végétaux et plantes entières.**

(57) Le substrat peut être utilisé aussi bien pour la culture in vitro qu'à l'extérieur ou sous serre ou pour la culture hydroponique horizontale ou verticale.

Ce substrat est caractérisé en ce qu'il est constitué de fibres synthétiques (2) inertes chimiquement et microbiologiquement.

EP 0 117 766 A1

0117766

La présente invention concerne un substrat de culture pour les cellules, tissus, organes végétaux et plantes entières utilisable aussi bien pour la culture in vitro en conditions stériles qu'à l'extérieur ou sous serre en conditions de culture hydroponique habituelle horizontale, en condition de culture hydroponique verticale, ou bien encore comme additif aux substrats de culture traditionnels.

La culture in vitro se réalise généralement sur des milieux gélifiés et sucrés. L'un des buts de cette culture in vitro est la multiplication végétative accélérée de plantes d'un certain intérêt économique. Cette multiplication végétative passe par un certain nombre d'étapes: prolifération et fractionnement des bourgeons, enracinement et sortie du jeune plant hors du récipient de culture, repiquage sur le substrat définitif et acclimatation aux conditions extérieures.

L'enracinement et la sortie du plant du récipient stérile sont souvent des étapes délicates. En effet les milieux gélifiés, asphyxiants par nature, ne sont jamais très favorables au bon développement du système racinaire. Par ailleurs, lors de la sortie du plant, il est nécessaire de laver abondamment le système racinaire pour le débarrasser du milieu gélifié sucré, entraînant ainsi des frais de main d'oeuvre importants et des risques de blessure du jeune plant. Le plant doit être repiqué à racines nues avant son acclimatation à l'ambiance extérieure. Un autre inconvénient présenté par la culture in vitro réalisée sur des milieux gélifiés est le coût élevé de l'opération qui nécessite, entre autres, l'utilisation de produits coûteux dont la gélose (ou Agar-Agar) qui entrent pour une part non négligeable dans le coût global de l'opération.

Dans le cas d'une culture à l'extérieur ou sous serre, dans des conditions de culture normale, on utilise habituellement des substrats composés de mélanges de tourbe et de terre dont les compositions sont rarement identiques d'un échantillon à l'autre et qui sont souvent contaminées par des microorganismes ou des graines étrangères.

2

Cependant, la culture des plantes horticoles ou maraîchères se fait de plus en plus en conditions de culture hydroponique.

Celle-ci présente, sur la culture traditionnelle, un certain nombre d'avantages à savoir un support propre, souvent stérilisable; une alimentation continue de la plante et un dosage précis des solutions nutritives.

Ceci se traduit en général par des améliorations de rendement et de précocité. Les supports employés sont de natures variables tels que sable, gravier, argile expansée, perlite, vermiculite, ou récemment de fibres minérales agglomérées ou en vrac. Ces dernières, de composition constante et inerte, sont, par contre, de contact désagréable, les fibres fines plus ou moins rigides perçant facilement la peau et occasionnant éventuellement des démangeaisons.

Tous ces supports sont, en général, d'origine minérale. Ils se présentent sous forme de particules séparées ou de fibres courtes enchevêtrées et agglomérées en pains par un liant. Leur faible cohésion oblige à les poser directement sur le sol ou sur des tablettes. Le mode de culture se rapproche ainsi du mode de culture traditionnelle, les plantes étant cultivées les unes à côté des autres.

Une amélioration de ces systèmes a été proposée pour la culture des fraisiers, où le substrat est enfermé dans un conteneur cylindrique vertical, les plants étant repiqués dans le substrat à travers la paroi du conteneur. Ce système de culture hydroponique verticale utilise actuellement les substrats d'origine minérale déjà nommés ou organiques traditionnels (tourbes etc....)

Le manque de cohésion de ces substrats rend obligatoire l'utilisation de conteneurs solides et résistants, plus ou moins imperméables à l'eau et aux gaz.

D'autre part, un tassement se produit obligatoirement dans le bas de ces colonnes de culture, tendant à créer des hétérogénéités dans les conditions de culture.

La présente invention vise à remédier à ces divers inconvénients en procurant un substrat de culture particulièrement économique et pouvant être utilisé aussi bien pour la culture in vitro qu'à l'extérieur ou sous serre ou pour la culture hydroponique horizontale ou verticale.

A cet effet ce substrat de culture pour les cellules, tissus, organes végétaux et plantes entières est caractérisé en ce qu'il est constitué de fibres synthétiques inertes chimiquement et microbiologiquement.

A titre d'exemple on peut utiliser, pour constituer le substrat de culture suivant l'invention, des fibres acryliques, modacryliques, polyamide, polyester, polypropylène, tétrafluoroéthylène,ou tout autre fibre ayant dess qualités similaires, à l'exclusion de toutes fibres minérales telles que laine de verre, laine de roche, amiante.

Le substrat peut être réalisé sous la forme de tampons de fibres ou encore de flocons utilisés seuls ou en tant qu'additif inerte dans des mélanges, ou bien encore de mèches solides pour la culture hydroponique verticale.

Dans le cas où le substrat est soumis à un traitement thermique, les fibres ou flocons doivent avoir un point de ramollissement inférieur à la température de traitement, par exemple elles doivent pouvoir résister à une température de 120°C. Par contre dans le cas du bouturage direct les fibre peuvent avoir un point de ramollissement beaucoup plus bas: on peut utiliser, par exemple, des fibres en chlorure de polyvinyle.

Le substrat de culture suivant l'invention offre de nombreux avantages par rapport aux substrats connus.

En premier lieu, lorsqu'il est employé sous forme de tampons cylindriques, l'excellente capillarité des tampons de fibres synthétiques permet de n'utiliser qu'une quantité faible de liquide nutritif. De ce fait seule la partie basse du tampon est saturée de liquide, la partie haute, aérée, permettant un excellent développement racinaire. Par ailleurs lorsque les racines sont bien développées dans le tampon, il est possible de sortir le plant

4

sans déranger le système racinaire. Le lessivage du milieu sucré se fait simultanément à l'acclimatation par le passage sous brouillard.

Lorsque le substrat de culture est utilisé sous forme de flocons , les fibres synthétiques qui le constituent et qui assurent l'inertie chimique et microbiologique, ont un contact doux et non irritant avec la peau. La structure de l'ensemble du substrat en forme de flocon permet une manipulation aisée du matériau, aussi bien pour le remplissage de pots ou conteneurs, si le produit est utilisé pur, que pour son incorporation mécanique avec d'autres substrats s'il est utilisé dans des mélanges nécessitant une très bonne aération et une structure stable. La légèreté des flocons rend le transport du substrat facile. Par ailleurs on obtient une structure très aérée, favorable à l'enracinement et au développement des plantes, une rétention d'eau importante grâce à sa structure en granulés, et une évaporation très faible du milieu nutritif en surface due à un effet de "mulch".

Dans le cas où le substrat se présente sous la forme d'une mèche, pour la culture hydroponique verticale, il présente l'avantage d'être suffisamment solide pour pouvoir être suspendu et permettre de repiquer les plants les uns au dessus des autres. La culture se fait ainsi "au volume" et non plus "à la surface" si bien que la serre peut être utilisée au maximum de ses capacités. Ce système permet de cultiver un nombre très important de plantes par unité de volume.

Par ailleurs, la solidité de la mèche donne à l'enveloppe un rôle uniquement de maintien latéral, sans exiger d'elle de résistance à la traction. Ceci permet une enveloppe légère et très aérée sur une mèche de faible diamètre. On obtient ainsi une aération maximale de racines permettant un développement optimal des plantes. La mèche présente, d'autre part, une structure homogène sur toute sa longueur.

On décrira ci-après,à titre d'exemples non limitatifs, diverses formes d'exécution de la présente invention, en référence au dessin annexé sur lequel :

La figure 1 est une vue en perspective d'un substrat de culture réalisé sous la forme d'un tampon de fibres.

La figure 2 est une vue en coupe verticale schématique d'un substrat de culture réalisé sous la forme d'une masse de flocons de fibres utilisés seuls.

La figure 3 est une vue en coupe verticale schématique d'un substrat de culture constitué par un mélange de flocons de fibres synthétiques et d'un autre matériau.

La figure 4 est une vue en perspective d'un substrat de culture réalisé sous la forme d'une mèche pendant verticalement pour la culture hydroponique verticale.

La figure 5 est une vue en coupe d'une variante d'exécution dans laquelle le substrat de culture est porté par une plaque perforée.

La figure 6 est une vue en coupe longitudinale partielle d'une autre variante d'exécution du substrat de culture.

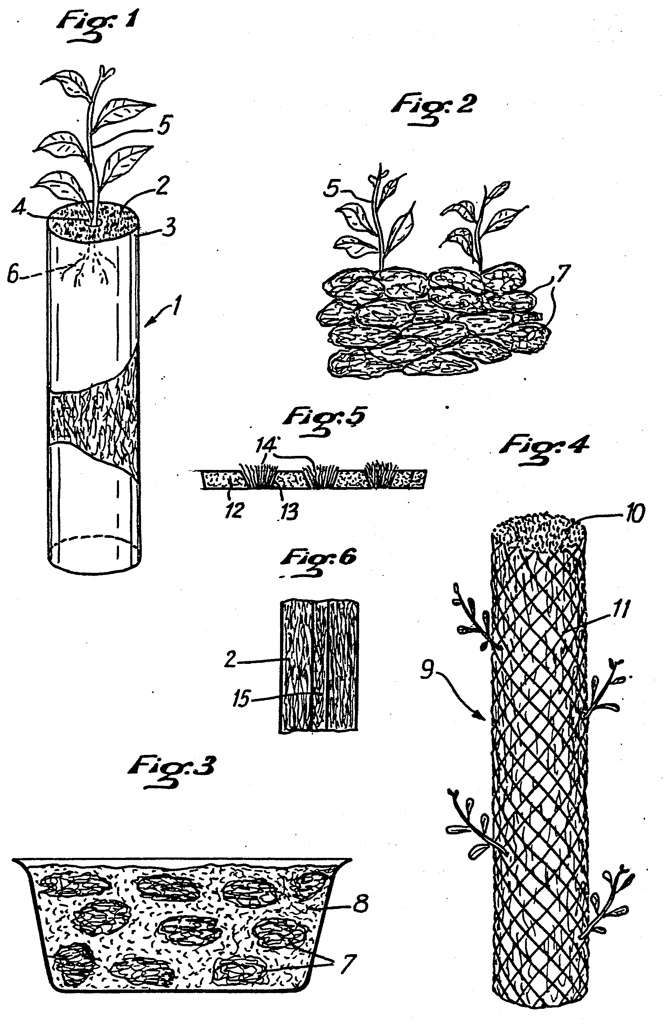

Sur la figure 1 est représenté un tampon 1 de forme cylindrique qui est composé uniquement de fibres synthétiques 2, inertes en milieu aqueux et résistant à l'autoclavage à 120°C telles que fibres acryliques, modacryliques, polyamide, polyester, polypropylène, tétrafluoéthylène, etc. Ces fibres sont assemblées en tampons de longueurs et diamètres variables suivant l'usage final. Les fibres 2 qui composent ces tampons 1, sont essentiellement des fibres longues, alignées, frisées ou non, continues ou discontinues, blanches ou colorées dans la masse. Le paquet de fibres synthétiques 2 est logé à l'intérieur d'une enveloppe cylindrique 3 qui peut être réalisée en papier fin, en textile tissé ou non ou encore en filet plastique extrudé.

Un avant-trou 4 peut être éventuellement pratiqué longitudinalement dans l'axe du tampon 1.

La densité des fibres 2 constituant la masse interne du tampon répond à deux conditions : l'espace doit être suffisamment réduit entre les fibres pour permettre une bonne capillarité de l'ensemble et par ailleurs il doit assurer une facilité de cheminement des racines entre les fibres. On a représenté sur la figure 1 un plant 5 dont les racines 6 se sont développées à l'intérieur du tampon 1, dans les interstices entre les fibres 2.

Les tampons 1 peuvent être ou non préimprégnés de substances de croissance et/ou d'éléments nutritifs nécessaires aux besoins des tissus végétaux ou fragments de plantes mis en culture.

Les tampons 1 peuvent être utilisés au laboratoire ou en tant que substrat de bouturage direct.

Dans le cas de l'utilisation en laboratoire, les tampons 1 sont introduits dans des tubes de culture ou installés sur un support ad hoc contenu dans le récipient de culture. Une petite quantité de milieu liquide de culture, calculée de façon qu'un tiers environ de la hauteur du tampon soit immergé, est introduite dans le récipient. L'ensemble est ensuite stérilisé à l'autoclave à 120°C.

L'excellente capillarité du tampon 1 permet l'établissement direct des cultures à partir de petits fragments végétaux tels que des méristèmes. Toutes les étapes de la culture in vitro peuvent être ainsi réalisées sur les tampons.

Le meilleur usage reste cependant le stade final de l'enracinement : les microboutures sont introduites stérilement dans l'avant trou 4 ménagé dans le tampon. Une fois l'enracinement effectué, l'ensemble plante et tampon est passé sous brouillard artificiel permettant simultanément l'acclimatation de la plante aux conditions extérieures et le lesivage du tampon des éléments tels que les sucres, qui pourraient être néfastes en conditions non stériles.

La pré-imprégnation possible du tampon par une solution nutritive simplifie encore les opérations, puisqu'il suffit alors, après avoir installé le tampon dans le récipient de culture, d'y ajouter une quantité d'eau purifiée, avant l'autoclavage de l'ensemble.

0117766

7

Un autre avantage de ce tampon réside dans le fait de permettre, en cours de culture, un renouvellement ou changement de milieu nutritif permettant d'adapter celui-ci aux exigences successives de la culture : bourgeonnement, enracinement, repos végétatif etc...

Dans le cas où le tampon 1 est utilisé en tant que substrat de bouturage direct, la bouture est introduite partiellement dans le tampon 1 à l'endroit du trou 4 préalablement formé ou en forçant l'écartement des fibres 2. L'apport des substances de croissance nécessaires à l'enracinement peut être donné soit par pré-imprégnation du tampon 1, soit par trempage des boutures dans une poudre ou un liquide contenant les substances nécessaires. L'ensemble bouture plus tampon est placé sous brouillard artificiel ou à l'étouffée.

Les racines se développent au sein du tampon et constituent une minimotte qui n'est pas dérangée lors des manipulations ultérieures. Ceci permet, pour les plantes délicates, un transfert facile sur le substrat définitif, sans provoquer d'arrêt de végétation. La plantation définitive ou la vente des jeunes plantes se fait au moment où les racines commencent à être visibles à l'extérieur du tampon.

La réserve d'eau maintenue dans le tampon 1 permet le transport ou le stockage momentané des jeunes plants sans qu'ils aient à souffrir de dessication.

L'inertie chimique et microbiologique des matériaux constituant ces tampons garantit, lors du bouturage, la non contamination par des germes pathogènes qui pourraient être contenus dans les substrats usuels tels que tourbe, terre.

Les figures 2 et 3 illustrent l'utilisation d'un substrat de culture suivant l'invention sous la forme de flocons 7. Chacun de ces flocons 7 est constitué de fibres synthétiques courtes, inertes, résistant à 120°C et agglomérées mécaniquement en flocons subsphériques d'environ 5 mm de diamètre. Une fois formés ces flocons gardent leur cohérence grâce à l'attraction électrostatique entre les fibres.

8

Les flocons de fibres synthétiques 7 peuvent être utilisés seuls et constituer une masse sur laquelle se développent les plants 5 comme il est représenté sur la figure 2. Ils forment alors dans ce cas, à eux seuls, des substrats de culture in vitro ou de culture extérieure, en tablettes ou conteneurs.

Les flocons 7 peuvent être également utilisés en tant qu'amendement de structure aux substrats classiques pour la culture (culture en pots ou tablettes, jardins, terrains de sport, etc).

La figure 3 illustre l'utilisation des flocons 7 comme additifs inertes mélangés à de la terre 8 pour assurer une bonne aération et une structure stable.

Les flocons 7 peuvent être également préimprégnés d'un gel hydrophile utilisé à l'état sec en mélange avec un substrat tel que la tourbe par exemple. Dans ce cas le substrat de culture réalisé sous la forme de flocons permet la réhydratation immédiate de la tourbe, réhydratation qui autrement serait longue et difficile.

La figure 4 illustre l'utilisation du substrat de culture suivant l'invention sous la forme d'une mèche 9 pour la culture hydroponique verticale. Cette mèche 9 est composée de fibres synthétiques 10, continues, non coupées, parallèles, enveloppées par une gaine ajourée 11, par exemple un filet plastique à mailles extrudées, de diamètre variable suivant les utilisations. La mèche 9 est suspendue par tout dispositif approprié et une solution nutritive est amenée goutte à goutte à l'extrémité supérieure de la mèche, cette solution descendant par gravité le long de la mèche en imbibant toutes les fibres. Les plantes sont repiquées dans la mèche 9 à travers la gaine ajourée 11. Une telle mèche 9 permet de cultiver un nombre très important de plantes par unité de volume et par ailleurs elle assure une aération des racines entraînant un développement optimal de la plante.

Le substrat de culture suivant l'invention peut être également constitué par une tresse plate ou circulaire constituée à partir de mèches de fibres continues, le nombre de mèches constituant la tresse n'étant pas limité. Cette tres-

9

se peut comporter ou non une mèche axiale imprégnée d'un gel hydrophile à l'état sec, s'imbibant et se gonflant en présence d'eau liquide.

Dans une variante d'exécution telle qu'illustré sur la figure 5 l'enveloppe du substrat de culture peut constituer le support direct de ce substrat de culture. Plus particulièrement ce support est constitué par une plaque 12, par exemple en polystyrène expansé ou en tout autre matière synthétique, cette plaque étant percée de trous 13 qui peuvent avoir avantageusement une forme conique. Dans chacun de ces trous 13 est logé un tampon de fibres 14 constituant un substrat de culture suivant l'invention. La plaque 12 peut être plane ou façonnée suivant tout modèle désiré.

Dans la forme d'exécution représentée sur la figure 6 le substrat de culture comprend, dans l'axe du tampon de fibres 2, une mèche longitudinale 15, constituée ou non du même matériau que celui des fibres 2 et qui est imprégnée d'un gel hydrophile à l'état sec, s'imbibant et se gonflant en présence d'eau liquide. Cette mèche 15 imprégnée de gel hydrophile peut être introduite longitudinalement au cours de la fabrication du tampon de fibres 2, dans la partie centrale de celui-ci. Cette mèche de gel hydrophile peut être également introduite dans le substrat de culture 9 représenté sur la figure 4. La mèche de gel 15 joue alors le rôle de réserve tampon de liquide permettant des arrosages non plus continus mais intermittents.

10

REVENDICATIONS

1 - Substrat de culture pour les cellules, tissus, organes végétaux et plantes entières caractérisé en ce qu'il est constitué de fibres synthétiques (2) inertes chimiqement et microbiologiquement.

2 - Substrat de culture suivant la revendication 1 caractérisé en ce qu'il est consitué de fibres acryliques, modacryliques, polyamide, polyester, polypropylène, tétrafluoroéthylène, ou tout autre fibre synthétique ayant des qualités similaires.

3 - Substrat de culture suivant l'une quelconque des revendications précédentes caractérisé en ce qu'il est réalisé sous la forme d'un tampon constitué de fibres longues, alignées, frisées ou non, continues ou discontinues, colorées dans la masse ou non.

4 - Substrat de culture suivant la revendication 3 caractérisé en ce que le paquet de fibres synthétiques ( 2) est logé à l'intérieur d'une enveloppe cylindrique (3) ou d'une autre forme géométrique, qui peut être réalisée en papier fin, en textile tissé ou non, en feuille plastique ou en filet plastique extrudé.

5 - Substrat de culture suivant l'une quelconque des revendications 3 et 4 caractérisé en ce qu'un avant-trou (4) est pratiqué longitudinalement dans l'axe du tampon (1).

6.- Substrat de culture suivant l'une quelconque des revendicationss 1 et 2 caractérisé en ce qu'il est réalisé sous la forme d'une mèche solide (9) pour la culture hydroponique verticale, cette mèche (9) étant composée de fibres synthétique (10), continues, non coupées, parallèles, enveloppées par une gaine ajourée (11).

7.- Substrat de culture suivant l'une quelconque des revendications 3 à 6 caractérisé en ce que le tampon de fibres (2,10) comporte, dans sa partie centrale, une mèche longitudinale (15) imprégnée d'un gel hydrophile à l'état sec, s'imbibant et se gonflant en présence d'eau liquide.

0117766

11

8 - Substrat de culture suivant l'une quelconque des revendications 1 et 2 caractérisé en ce qu'il est réalisé sous la forme de flocons(7) utilisés seuls ou en tant qu'additif inerte dans des mélanges avec des substrats classiques tels que terre, tourbe, etc.

9.- Substrat de culture suivant la revendication 8 caractérisé en ce que les flocons (7) sont imprégnés d'un gel hydrophile utilisé à l'état sec et mélangé avec un substrat classique tel que terre, tourbe, etc.

10.- Substrat de culture suivant l'une quelconque des revendications 1 et 2 caractérisé en ce qu'il est constitué de tampons de fibres (14) logées dans des trous (13) d'une plaque perforée (12) servant de support direct de ces tampons de fibres.

0117766

1/1

Fig. 1

Fig. 2

Fig. 5

Fig. 4

Fig. 6

Fig. 3

**Office européen
des brevets**

# RAPPORT DE RECHERCHE EUROPEENNE

Numéro de la demande

EP 84 40 0022

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| A | FR-A-2 095 402 (SOCIETE RHODIACETA) * Page 1, ligne 26 - page 2, ligne 36 * | 1,2 | A 01 G 31/02 A 01 G 9/10 |
| A | FR-A-2 442 809 (GUICHEBARON) * Revendication 1 * | 1 | |
| A | GB-A-1 562 182 (SANDERS) * Page 3, lignes 19-40; revendications 1,8,9 * | 1 | |
| A | FR-A-2 314 659 (TROPEA) * Page 3, ligne 4 - page 4, ligne 5; figures 1,2 * | 4,6 | |
| P,X | US-A-4 403 446 (WOLFE) * Colonne 3, ligne 50 - colonne 4, ligne 42; colonne 5, lignes 19-42 * | 1-3,6 | **DOMAINES TECHNIQUES RECHERCHES (Int. Cl. 3)** A 01 G |

Le présent rapport de recherche a été établi pour toutes les revendications

| Lieu de la recherche LA HAYE | Date d'achèvement de la recherche 12-04-1984 | Examinateur HERYGERS J.J. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82